# NEUE EUROPÄISCHE PATENTSCHRIFT

(11) **EP 1 185 291 B2**
(45) Veröffentlichungstag und Bekanntmachung des Hinweises auf die Entscheidung über den Einspruch: **27.10.2010**
(45) Hinweis auf die Patenterteilung: 04.02.2004
(21) Anmeldenummer: 00943666.8
(22) Anmeldetag: 26.05.2000
(51) Int. Cl.: A61K 38/16, A61K 8/00, A61P 25/00, C07K 14/33

(54) **THERAPEUTIKUM MIT EINEM BOTULINUM-NEUROTOXIN**
THERAPEUTIC AGENT COMPRISING A BOTULINUM NEUROTOXIN
AGENT THERAPEUTIQUE COMPORTANT DE LA BOTULINUM-NEUROTOXINE DE BOTULINUM

(30) Priorität: 07.06.1999 DE 19925739
(43) Veröffentlichungstag der Anmeldung: 13.03.2002
(73) Patentinhaber: Merz Pharma GmbH & Co. KGaA, 60318 Frankfurt (DE)
(72) Erfinder: BIGALKE, Hans, D-30419 Hannover (DE); FREVERT, Jürgen, D-14473 Potsdam (DE)
(74) Vertreter: Krauss, Jan
(86) Internationale Anmeldenummer: PCT/DE2000/001777
(87) Internationale Veröffentlichungsnummer: WO 2000/074703

(56) Entgegenhaltungen:
- WO-A-94/28923
- WO-A-96/39167
- AU-B2- 200 058 047
- DE-A- 19 856 897
- US-A- 5 512 547
- US-A- 5 939 070
- GOESCHEL HILKE ET AL: "Botulinum A toxin therapy: Neutralizing and nonneutralizing antibodies-therapeutic consequences." EXPERIMENTAL NEUROLOGY, Bd. 147, Nr. 1, 1997, Seiten 96-102, XP002158434 ISSN: 0014-4886
- EDITORIAL: "botulinum toxin" THE LANCET, Bd. 340, Nr. 19/26, Dezember 1992 (1992-12), Seiten 1508-1509, XP002158435
- KEEN M ET AL: "Botulinum Toxin A for Hyperkinetic Facial Lines: Results of a Double-Blind, Placebo-Controlled Study" PLASTIC AND RECONSTRUCTIVE SURGERY, Bd. 94, Nr. 1, Juli 1994 (1994-07), Seiten 94-99, XP000982177
- NAUMANN M ET AL: "Depletion of neutralising antibodies resensitizes a secondary non-responder to botulinum A neurotoxin." JOURNAL OF NEUROLOGY NEUROSURGERY AND PSYCHIATRY, Bd. 65, Nr. 6, Dezember 1998 (1998-12), Seiten 924-927, XP000979025 ISSN: 0022-3050
- Wohlfahrt, K. et al.; In Tranter HS, ed. Proceedings of Biomedical Aspects of Clostridial Neurotoxins Intl. Conf. Oxford, July 7-11, 1996,Porton Down, Salisbury, Wiltshire SP4 0JG, UK
- GREENE, P. E. ET AL. MOV. DISORD. Bd. 8, Nr. 4, 1993, Seiten 479 - 483
- CHEN, R. ET AL. NEUROLOGY Bd. 51, November 1998, Seiten 1494 - 1496
- HANNA, PHILIP A. ET AL. J. NEUROL. NEUROSURG. PSYCHIATRY Bd. 66, 1999, Seiten 612 - 616
- LEW, M. F. ET AL. NEUROLOGY Bd. 49, 1997, Seiten 701 - 707
- Merz Pharmaceuticals GmbH, 2005

## Beschreibung

Die vorliegende Erfindung betrifft die Verwendung von Botulinum-Neurotoxinen von *Clostridium botulinum* zur Herstellung eines Medikaments zur Behandlung von Erkrankungen des Nervensystems, wobei das Neurotoxin frei ist von den komplexierenden Proteinen, die natürlicherweise in dem Komplex vorliegen. Die unmittelbare Folge davon ist die der vorliegenden Erfindung zu Grunde liegende Erkenntnis, dass das freie Neurotoxin im Gegensatz zum Komplex im Patienten zu keiner oder nur zu einer deutlich verminderten Induktion neutralisierender Antikörper führt. Ein anderer Aspekt der vorliegenden Erfindung betrifft die Verwendung der Botulinum-Neurotoxine von *Clostridium botulinum* für die kosmetische Behandlung.

*Clostridium botulinum* Toxinkomplex Typ A (Mᵣ 900.000) wird seit mehreren Jahren zur Therapie verschiedener Dystonien eingesetzt. Derzeit sind zwei verschiedene, diesen Komplex enthaltende Präparate für die Behandlung des *Blepharospasmus,* hämifacialer Spasmen und des *Torticollis spasmodicus* zugelassen: BOTOX® und DYSPORT®. Die Therapie weiterer Erkrankungen des Nervensystems (z. B. Spastizitäten, Migräne, Lumbalgie, Cervicalsyndrom, Hypersalivation) wird derzeit klinisch geprüft. Die Präparate werden außerdem bei kosmetischen Indikationen wie Hyperhidrose und ausgeprägter Faltenbildung eingesetzt. Auch die übrigen *Clostridium botulinum* Toxinkomplexe (der Typen B, C, D, E, F, G) eignen sich für diese Therapien. Allerdings gibt es derzeit noch kein zugelassenes Produkt auf dem Markt, das Einsender Toxine vom Typ B-G enthält.

Botulinumtoxin-Komplexe setzen sich aus einem Gemisch clostridieller Proteine zusammen. Dies sind Hämagglutinine mit unterschiedlichen Molekularmassen, ein nicht-toxisches nichthämagglutinierendes Protein (Mᵣ ca. 120.000) und ein Neurotoxin (Mᵣ ca. 150.000). Sie bilden einen säurestabilen Komplex, der für die orale Toxizität bei Lebensmittelintoxikationen verantwortlich ist. Im Gegensatz zum reinen Neurotoxin widersteht der Komplex dem agressiven Milieu im Magen-Darm-Trakt und ermöglicht die enterale Resorption des Neurotoxins, welches über den Blutkreislauf oder das Lymphsystem die Zielzellen erreicht und dort eine Blockade der Transmitterfreisetzung auslöst. Daraufhin kommt es zur Paralyse der quergestreiften und glatten Muskulatur und zum Versiegen verschiedener vegetativer Funktionen. Vergiftete Patienten sterben an einer Insuffizienz der respiratorischen Muskulatur. Da das reine Neurotoxin im Magen-Darm-Trakt abgebaut und somit nicht enteral resorbiert wird, ist es nach einer Ingestion nicht giftig. Parenteral appliziert unterscheiden sich die therapeutischen Wirkungen des Neurotoxins und des Komplexes nicht denn der Komplex zerfällt im Gewebe in seine Bestandteile, und nur das Neurotoxin wird in die Zielzellen aufgenommen.

Zur therapeutischen Anwendung wird der Komplex nach dem heutigen Stand der Technik direkt in dystone oder spastische Muskel injiziert, wo das Neurotoxin bei physiologischem pH aus dem Komplex freigesetzt wird und die erwünschte pharmakologische Wirkung hervorruft. Obwohl der Komplex nur in äußerst geringen Dosen appliziert wird (1-25 ng, je nach Indikation und Größe des betroffenen Muskels), kommt es nach wiederholten Injektionen bei einer beträchtlichen Anzahl der Patienten zur Bildung von spezifischen, neutralisierenden Antikörpern, die auch gegen das Neurotoxin gerichtet sind. Unmittelbare Folge ist, dass Antikörper-positive Patienten auf den Komplex nicht mehr ansprechen. Sie könnten aber mit anderen Toxintypen behandelt werden, von denen allerdings keiner zur Therapie zugelassen ist. Wenn der Patient alle Toxin-Typen durchprobiert und Antikörper gegen sie gebildet hat, ist die weitere Applikation eines Botulinumtoxin-Komplexes (egal welchen Typs) nicht mehr hilfreich. Dabei ist zu berücksichtigen, dass jede Gabe des Komplexes zu einer Erhöhung des Antikörper-Titers beiträgt bis zu dem Punkt, an dem eine weitere Applikation des Komplexes keinen Sinn mehr macht, weil kein Effekt mehr erzielt wird. Bis der Antikörper-Titer nennenswert gesunken ist, vergehen oft Jahre, so dass diese Patienten über lange Zeiträume nicht (mit Botulinum-Neurotoxin) behandelt werden (können).

Die Bildung von spezifischen Antikörpern wird durch zwei Faktoren begünstigt. Zum einen bleibt das Neurotoxin, fixiert im Komplex, über einen längeren Zeitraum im Gewebe liegen und kann ins Gewebe wandernde Immunzellen zur Antikörperbildung aktivieren. Die lange Verweildauer führt jedoch zu keiner gesteigerten Aufnahme in die Zielzellen, denn vergiftete Zielzellen können kein Toxin mehr aufnehmen. Das langsam aus dem Komplex herausdissoziierende Neurotoxin ist also nur noch immunologisch wirksam. Zum anderen verstärken die im Komplex enthaltenen Proteine eine Immunantwort. Hämagglutinine sind Lektine, also Proteine, die sich durch eine hohe Affinität zu bestimmten Zuckern auszeichnen. Aufgrund ihrer Bindung an Zuckerstrukturen wirken Lektine immunstimulierend. So konnte gezeigt werden, daß die Lektine Concanavalin A, Phytohämagglutinin und Pokeweed Mitogen die T- und B-Lymphozyten aktivieren. Die Hämagglutinine der Botulinumtoxin-Komplexe, die ebenfalls an membranständige Zucker binden, können also in ähnlicher Weise als Immunadjuvanzien fungieren und zur Antikörperbildung und damit zum Therapieversagen beitragen.

Es wurde gezeigt, daß die Antigenizität von Botulinum-Neurotoxin des Typs A mit dem Reinheitsgrad des Toxin abnimmt (US-A-5512547). Da die komplexierenden Proteine als Adjuvans wirken, die die Produktion von anti-Neurotoxin Antiköpern stimulieren, wurde erkannt daß die nächste Generation von Botulinum-Neurotoxin frei von komplexierenden Proteinen sein sollte. Vor der Entwicklung der vorliegenden Erfindung bestand jedoch erheblicher Zweifel, ob eine Behandlung von Patienten mit bereits existierender Immunität gegen Botulinum-Neurotoxin mit solchen Präparaten möglich sei (Goeschel, H. et al.: Experimental Neurology 147, No.1, 1997, pages 96-102).

Deswegen stellte sich für die Erfinder der vorliegenden Erfindung die Aufgabe, einen alternativen Behandlungsweg für oben genannte Erkrankungen und Störungen zu entwickeln. Insbesondere wollten die Erfinder einen geeigneten Wirkstoff vorschlagen, mit dem Patienten, die bereits neutralisierende Antikörper gebildet haben, thenpiert werden können.

Als Lösung der oben gestellten Aufgabe, als Alternative zu den beiden kommerziellen Präparaten aus Botulinumtoxin-Komplex des Typs A, BOTOX® und DYSPORT® und auch als Alternative zu den im Stand der Technik beschriebenen Komplexen der übrigen Typen (B, C, D, E, F, G), wurde ein neues Arzneimittel entwickelt, welches nur reines Neurotoxin (Typ A bzw. B, C, D, B, F, G) enthält und frei von Hämagglutininen und anderen körperfremden Proteinen ist. Wegen seiner geringeren Molekularmasse diffundiert es schneller zu den Zielzellen, in die es aufgenommen wird, bevor Immunzellen, von Hämagglutininen angelockt, aktiviert werden. In Antigenitätsstudien fanden wir, daß das reine Neurotoxin aller Typen - im Unterschied zu kommerziellen Präparaten des Typs A und den Komplexen der Typen B bis G keine oder allenfalls eine sehr geringe Bildung von Antikörpern auslöst. Bei therapeutischem Einsatz dieser neu entwickelten Arzneimittel (reines Neurotoxin der Typen A, B, C, D, E, F, G) kommt es auch nach wiederholten Applikationen zu keinem Antikörper-bedingten Therapieversagen. Ferner konnte gezeigt werden, daß sich die reinen Neurotoxine wegen ihrer sofortigen Bioverfügbarkeit auch weiterhin zur Therapie von Patienten eignen, die nach Applikation eines Botulinumtoxin-Komplexes, z.B. nach Behandlung mit BOTOX® oder DYSPORT®, einen Antikörpertiter gegen den entsprechenden Typ entwickelt haben (sog. secondary non-responders), also einer weiteren Behandlung mit BOTOX® oder DYSPORT® nicht mehr zugänglich sind, da die Verabreichung der kommerziellen Toxine keine Linderung der Beschwerden mehr bringt.

Das erfindungsgemäß bereitgestellte Arzneimittel eignet sich als Therapeutikum besonders bei Patienten, die einen Antikörper-Titer gegen ein Botulinum-Toxin, insbesondere gegen das des Typs A, ausweisen. Besonders geeignet ist das erfindungsgemäße Pharmazeutikum (reines Neurotoxin oder Gemisch von mehreren reinen Neurotoxinen) bei solchen Patienten, die einen Antikörper-Titer von nicht größer als 50, bevorzugt nicht größer als 30, mehr bevorzugt nicht größer als 20, besonders bevorzugt nicht mehr als 10, und ganz besonders bevorzugt nicht mehr als 5 mU/ml aufweisen. Dabei ist 1 mU Antikörper diejenige Menge an Antikörper, die 10 U Toxin neutralisiert.

Andererseits kann das erfindungsgemäße Arzneimittel besonders vorteilhafterweise bei solchen Personen eingesetzt werden, die noch nie zuvor, oder schon lange Jahre nicht mehr, mit Botulinum-Neurotoxin behandelt worden sind, da deren Antikörper-Titer von Anfang an niedrig oder gleich Null ist. Das Vorteilhafte an der vorliegenden Erfindung besteht dann darin, dass der Titer dieser Patienten durch die Behandlung mit dem reinen Toxin gemäß der vorliegenden Erfindung nicht, oder allenfalls sehr unwesentlich, erhöht wird. Mit anderen Worten, das erfindungsgemäße Therapeutikum kann über lange Zeiträume verabreicht werden, ohne dass es seine Wirkung einbüßt.

Die Induktion von Antikörpern bei der Therapie mit einem *C. botulinum* Toxin wird also dadurch verhindert, dass statt der hochmolekularen toxischen Komplexe ein reines Neurotoxin appliziert wird. Das von den Komplexproteinen vollständig getrennte Neurotoxin ist sofort bioverfügbar und kann unmittelbar an die Nervenendigungen der motorischen Endplatten binden.

Ein Aspekt der vorliegenden Erfindung ist die Verwendung der Botulinum-Neurotoxine von *Clostridium botulinum* der Typen A, B, C, D, E, F, oder G oder eines Gemisches von zwei oder mehr dieser Neurotoxine, dadurch gekennzeichnet, daß das Neurotoxin bzw. das Gemisch der Neurotoxine frei ist von den komplexierenden Proteinen, die auf natürlicher Weise Komplexe mit den Botulinum-Neurotoxinen bilden, zur Herstellung eines Pharmazeutikums zur kosmetischen Behandlung oder zur Behandlung von Dystonien oder von Erkrankungen des Nervensystems in tierlichen oder menschlichen Patienten, die bereits neutralisierende Antikörper gegen Botulinum-Neurotoxin Komplexe gebildet haben.

Solche Pharmazeutika koennen vorteilhaft benutzt werdem, um Patienten zu behandeln, die bereits neutralisierende Antikörper gegen einen Komplex von *Clostridium botulinum* Typ A oder B, oder einen Komplex von *Clostridium botulinum* Typ A und B aufweisen.

Diese Pharmazeutika eignen sich besonders für die kosmetische Behandlung von Hyperhidrose und/oder Faltenbildung, z.B. im Faltenbildung im Gesichtsbereich.

Zu den Erkrankungen des Nervensystems und Dystonien welche mit diesen Pharmazeutika behandelt werden koennen zählen Torticollis spasmodicus und Blepharospasmus, Spastizitäten wie Spitzfuß, hämifasciale Spasmen, Migräne, Lumbalgie, Cervicalsyndrom und Hypersalivation.

Die Neurotoxine, ihre Gemische bzw. die erfindungsgemäßen pharmazeutischen Zubereitungen können als wässrige Lösung, insbesondere als wässrige Injektionslösung, aber auch als lyophilisierte Produkte vorliegen.

Die an sich bekannten reinen Neurotoxine der Typen A-G wurden hergestellt nach den Protokollen, die in den in der Literaturliste aufgeführten Veröffentlichungen enthalten sind. Beispielhaft ist die Aufreinigung von zwei Neurotoxinen (Typ A und B) in den nachfolgenden Beispielen beschrieben.

### Beispiel 1: Isolierung des reinen Neurotoxins

Das reine Neurotoxin aus *Clostridium botulinum* Typ A wird nach einem Verfahren gewonnen, das sich an das Verfahren von DasGupta & Sathyamoorthy anlehnt. *Clostridium botulinum* Typ A wird in einem 20-1-Fermenter in einem Medium kultiviert, das aus 2% Proteose Peptone, 1% Yeast-Extrakt, 1% Glukose und 0.05% Natriumthioglycolat besteht. Nach 72 Stunden Wachstum wird das Toxin durch Zugabe von 3N H₂SO₄ (End-pH = 3.5) gefällt. Die präzipitierte und zentrifugierte Biomasse wird mit 0.2 M Natriumphosphatpuffer pH 6.0 extrahiert.

Nach Abtrennung der Nukleinsäuren durch Fällung mit Protaminsulfat wird das Toxin durch Zugabe von Ammoniumsulfat gefällt. Das solubilisierte und gegen 50 mM Natriumphosphat pH 6.0 dialysierte Präzipitat wird auf einer DEAE-Sephadex-Säule beim gleichen pH-Wert gebunden und mit 150 mM NaCl abgelöst. Anschließend erfolgt eine Chromatographie über eine QAE-Sephadex-Säule, die mit einem 50 mM Tris/HCl-Puffer pH 7.9 äquilibriert wurde. Das Toxin wird über einen NaCl-Gradienten eluiert. Im letzten Schritt wird das Toxin über SP-Sephadex bei pH 7.0 chromatographiert. Das gebundene Toxin wird dabei mittels eines NaCl-Gradienten (0 - 300 mM) von der Säule abgelöst. Das gereinigte Toxin wird in einer SDS-Polyacrylamidgelelektrophorese (SDS-PAGE) analysiert und weist eine Reinheit von 95 ± 5 % auf. Die biologische Aktivität wird im Maus LD₅₀-Assay bestimmt: einer LD₅₀-Einheit entsprechen 4.8 pg Protein.

### Beispiel 2: Herstellung eines Botulinum-Neurotoxin-haltigen Fertigarzneimittels

Mit dem gereinigten Neurotoxin aus Beispiel 1 wird eine Lösung hergestellt, die 200 Maus-LD₅₀-Einheiten, 10 mg Saccharose und 2 mg humanes Serumalbumin pro ml enthält. 0.5 ml der Lösung werden in Vials abgefüllt und gefriergetrocknet. Die Lyophilisate werden mit physiologischer Kochsalzlösung rekonstituiert und die biologische Aktivität bestimmt. Die Vials enthalten 100 ± 30 LD₅₀-Einheiten.

### Beispiel 3: Isolierung von reinem Neurotoxin B

*Clostridium botulinum* Typ B wird im gleichen Medium und unter gleichen Bedingungen wie Typ A kultiviert und bis zur Ammoniumsulfatfällung aufgearbeitet. Anschließend erfolgt wiederum eine DEAE-Sephadex-Chromatographie bei pH 6.0. Die mit 150 mM NaCl von der Säule eluierten Fraktionen werden vereinigt und gegen Natriumphosphat pH 7.0 dialysiert, woran sich eine Chromatographie über QAE-Sephadex anschließt. Die toxinhaltigen Fraktionen werden weiter über eine DEAE-Sephadex-Chromatographie bei pH 8.5 (50 mM Tris/HCl pH 8.5) chromatographiert.

Schließlich erhält man das hochreine Botulinumtoxin Typ B über eine Chromatographie an Hydroxylapatit äquilibriert mit 10 mM Na-Phosphat pH 8.0. Das gebundene homogene Toxin wird mit 80 mM Na-Phosphat pH 8.0 eluiert und anschließend die biologische Aktivität im LD₅₀-Assay bestimmt (2 - 4 x 10⁷ LD₅₀-Einheiten/mg Protein).

### Beispiel 4: Nachweis von Antikörpern

20 Kaninchen wurden 25 U BOTOX® über einen Zeitraum von 12 Wochen im Abstand von 14 Tagen (5 Injektionen) intracutan injiziert. Nach 3 Wochen und dann im Abstand von 14 Tagen wurde Serum gewonnen.

Antikörper gegen *Clostridium botulinum* Neurotoxin A wurden mit einem Enzymimmunoassay nachgewiesen, indem das homogene Neurotoxin auf einer Mikrotiterplatte immobilisiert war. An das Neurotoxin bindende Antikörper wurden mittels eines zweiten, enzymmarkierten Antikörpers quantifiziert.

Das Ergebnis ist in Tabelle 1 dargestellt. Bereits 5 Wochen nach der ersten Applikation konnten bei 5 Kaninchen Antikörper nachgewiesen werden. Nach 11 Wochen enthielten Seren von 17 Kaninchen, also 85% der eingesetzten Tiere, Antikörper gegen das Neurotoxin Im biologischen Aktivitätstest wurde gezeigt, daß 12 der 17 Seren neutralisierende Antikörper enthielten (Tabelle 2).

**Tab. 1**

| Bestimmung von Serumproben (1:100 verdünnt) aus Kaninchen, die mit BOTOX® behandelt wurden mit einem Enzym-Immunoassay. OD₄₉₀ₙₘ > 0.1 sind angegeben. Alle OD-Werte sind korrigiert mit den OD-Werten der Präimmunseren (OD ca. 0,150). | | | | | |
|---|---|---|---|---|---|
| Kaninchen Nr. | 3. Woche | 5. Woche | 7. Woche | 9. Woche | 11. Woche |
| 1 | - | - | - | 0,11 | 0,36 |
| 2 | - | - | - | 2,36 | 2,23 |
| 3 | - | - | 0,57 | 1,43 | 1,44 |
| 4 | - | - | 0,68 | 1,68 | 0,93 |
| 5 | - | 0,97 | 3,52 | 3,49 | 3,44 |
| 6 | - | - | 1,34 | 2,32 | 2,70 |
| 7 | - | - | 2,13 | 3,09 | 3,00 |
| 8 * | - | 0,53 | 1,47 | 2,75 | 2,75 |
| 9 | - | - | 0,43 | 2,44 | 2,85 |
| 10 | - | - | 2,99 | 3,15 | 2,73 |
| 11 | - | 0,10 | 2,42 | 2,45 | 1,93 |
| 12 | - | - | - | 1,13 | 1,95 |
| 13 | - | - | - | - | 1,89 |
| 14 | - | - | - | - | - |
| 15 | - | - | - | - | - |
| 16 | - | - | - | - | - |
| 17 | - | 2,93 | 3,62 | 3,72 | 3,44 |
| 18 | - | - | 1,18 | 2,28 | 2,62 |
| 19 | - | - | 0,43 | 0,43 | 0,81 |
| 20 | - | 1,65 | 3,20 | 2,97 | 2,88 |

| | | | | | |
|---|---|---|---|---|---|
| * Die Werte wurden nicht korrigiert, da kein Präimmunserum vorhanden war "-" bedeutet Optische Dichte (OD₄₉₀) < 0,1 | | | | | |

**Tab. 2**

| Neutralisation durch Seren von Kaninchen, die mit BOTOX® (Woche 11 nach der ersten Immunisierung) im Maus-Hemidiaphragma-Assay (Nachweisgrenze: 0,35 mU/ml Antikörper) | |
|---|---|
| Kaninchen | Neutralisation mU/ml |
| 1 | 2,0 |
| 2 | n. d. |
| 3 | n. d. |
| 4 | > 10 |
| 5 | > 100 |
| 6 | n. d. |
| 7 | >10 |
| 8 | >10 |
| 9 | n. d. |
| 10 | n. d. |
| 11 | n. d. |
| 12 | > 10 |
| 13 | n. d. |
| 14 | n. d |
| 15 | < 0,35 |
| 16 | 0,4 |
| 17 | > 10 |
| 18 | > 10 |
| 19 | 2,0 |
| 20 | > 10 |
| n. d. = nicht bestimmt | |

### Beispiel 5: Antigenizitätstest mit Marktprodukt und reinem Neurotoxin

Nachdem gezeigt wurde, daß der Komplex aus Neurotoxin und Hämagglutininen und dem nichttoxischen, nicht-hämagglutinierenden Protein die Bildung neutralisierender Antikörper auslöst, wurde die immunogene Wirkung des reinen Neurotoxins (Typ A) geprüft. Dazu wurden 8 Kaninchen mit dem Toxinkomplex und 12 Kaninchen mit dem reinen Toxin behandelt. Nach dem oben beschriebenen Verfahren (siehe Beispiel 1) wurden 25 U des jeweiligen Präparates intracutan appliziert. Die Menge an Neurotoxin, gemessen als Gewicht, war in beiden Präparaten gleich (200 pg/Dosis), wie in einem ELISA nachgewiesen wurde. BOTOX® enthielt zusätzlich noch Komplexproteine (ca. 800 pg/Dosis).

Vier der acht mit BOTOX® behandelten Tiere zeigten im ELISA einen Antikörpertiter, während bei den 12 mit reinem Neurotoxin behandelten Tieren keine Antikörper gegen das reine Neurotoxin nachzuweisen waren. Das Ergebnis wurde im biologischen Aktivitätstest bestätigt. Alle vier Kaninchenseren enthielten neutralisierende Antikörpertiter, die eine Toxinwirkung verhinderten (Tabelle 3).

**Tab. 3**

| Neutralisation durch Seren (1:3 verdünnt) von Kaninchen, die mit BOTOX® (Woche 11 nach der ersten Immunisierung) im Maus-Hemidiaphragma-Assay (Nachweisgrenze: 1 mU/ml Antikörper) | |
|---|---|
| Kaninchen | Neutralisation mU/ml |
| 1 | 12 mU |
| 2 | > 30 mU |
| 3 | 4.5 mU |
| 8 | >30mU |

### Beispiel 6: (Vergleichsbeispiel)

In diesem Experiment wurde die Antikörperbildung durch BOTOX® mit der durch DYSPORT ® verglichen. Hierzu wurden jeweils zehn Kaninchen entweder mit BOTOX® (Gruppe 1), mit DYSPORT® (Gruppe 2) oder mit dem reinen Neurotoxin (Gruppe 3) nach beschriebenem Schema behandelt.

Während in Gruppe 1 und 2 mehr als 50% der Tiere einen neutralisierenden Antikörpertiter bildeten, waren die Seren aus den Tieren der Gruppe 3 frei von Antikörpern.

### Beispiel 7: Klinischer Test

Ein Patient (Alter 45 Jahre), der über einen Zeitraum von 5 Jahren wegen eines *Torticollis spasmodicus* mit BOTOX® behandelt wurde, hatte eine Antikörpertiter von 3 mU/ml Serum entwickelt. Weder BOTOX® noch DYSPORT® waren bei diesem Patienten therapeutisch wirksam. Ein Therapieversuch mit dem reinen Botulinum-Neurotoxin in einer Dosis von 145 U, welche äquivalent der zuletzt injizierten Dosis von BOTOX® war, führte innerhalb von 72 Stunden zur Lockerung des Muskels, zur Normalisierung der Kopfhaltung und zum Verschwinden des Muskelschmerzes. Unerwünschte Wirkungen traten nicht auf.

### Beispiel 8: Klinischer Test

Ein Patient (Alter 52 Jahre) wurde 3 Jahre wegen Cerebralparesee mit BOTOX® behandelt. Er hatte einen Antikörpertiter von 1 mU/ml Serum entwickelt, die Therapie mußte deshalb abgebrochen werden. Die Injektion von 200 U reinen Neurotoxins ermöglichte eine erfolgreiche Therapie.

### Literatur

DasGupta, B.R. & Sathyamoorthy, V. (1984), Purification and Amino Acid Composition of Type A Botulinum Neurotoxin; Toxicon 22(3), p. 415 - 424

De Jongh, K. S., Schwartzkoff, C. L. & Howden, M. E. H. (1989), Clostridium botulinum Type D Neurotoxin Purification and Detection; Toxicon 27 (2), p. 221 - 228

Schmidt, J. J. & Siegel, L. S. (1986), Purification of Type E Botulinum Neurotoxin by High-Performance Ion Exchange Chromatography; Analyt. Biochemistry 156, p. 213 - 219

Nukina, M., Mochida, Y., Sakaguchi, S. & Sakaguchi, G. (1988), Purification of Clostridium botulinum Type G Progenitor Toxin; Zbl. Bakt. Hyg. A 268, p. 220 - 227

Terajima, J., Syuto, B., Ochanda, J. O. & Kubo, S. (1985), Purification and Characterization of Neurotoxin Produced by Clostridium botulinum Type C 6813; Infection and Immunity 48 (2), p. 312 - 317

Wadsworth, J. D. F., Desai, M., Tranter, H. S. et al. (1990), Botulinum type F neurotoxin: Largescale Purification and Characterization of its Binding to Rat Cerebrocortical Synaptosomes; Biochem. J. 268, p. 123 - 128

## Patentansprüche

1. Verwendung der Botulinum-Neurotoxine von *Clostridium botulinum* der Typen A, B, C, D, E, F oder G oder eines Gemisches von zweien oder mehreren dieser Neurotoxine, **dadurch gekennzeichnet, dass** das Neurotoxin bzw. das Gemisch der Neurotoxine frei ist von den komplexierenden Proteinen, die auf natürliche Weise Komplexe mit den Botulinum-Neurotoxinen bilden, zur Herstellung eines Pharmazeutikums zur Behandlung von Dystonien oder von Erkrankungen des Nervensystems in tierischen oder menschlichen Patienten, die bereits neutralisierende Antikörper gegen Botulinum-Neurotoxin Komplexe gebildet haben.

2. Verwendung eines Pharmazeutikums umfassend Botulinum-Neurotoxine von *Clostridium botulinum* der Typen A, B, C, D, E, F oder G oder eines Gemisches von zweien oder mehreren dieser Neurotoxine, **dadurch gekennzeichnet, dass** das Neurotoxin bzw. das Gemisch der Neurotoxine frei ist von den komplexierenden Proteinen, die auf natürliche Weise Komplexe mit den Botulinum-Neurotoxinen bilden, zur kosmetischen Behandlung in tierischen oder menschlichen Patienten, die bereits neutralisierende Antikörper gegen Botulinum-Neurotoxin Komplexe gebildet haben.

3. Verwendung nach Anspruch 1 oder 2, **dadurch gekennzeichnet, dass** die zu behandelnde Person bereits neutralisierende Antikörper gegen einen Komplex von *Clostridium botulinum* Typ A oder B oder einen Komplex von *Clostridium botulinum* Typ A und B aufweist.

4. Verwendung nach Anspruch 2 oder 3, **dadurch gekennzeichnet, dass** die kosmetische Behandlung zur Behandlung der Hyperhydrose erfolgt.

5. Verwendung nach einem der Ansprüche 2 bis 4, **dadurch gekennzeichnet, dass** die kosmetische Behandlung zur Behandlung von Faltenbildung erfolgt.

6. Verwendung nach Anspruch 5, **dadurch gekennzeichnet, dass** die kosmetische Behandlung zur Behandlung von Faltenbildung im Gesichtsbereich erfolgt.

7. Verwendung nach Anspruch 1 oder 3, **dadurch gekennzeichnet, dass** es sich bei den Erkrankungen des Nervensystems bzw. den Dystonien um Torticollis spasmodicus, Blepharospasmus, Spastizitäten wie Spitzfuß, hämifasciale Spasmen, Migräne, Lumbalgie, Cervicalsyndrom oder Hypersalivation handelt.

## Claims

1. Use of the botulinum-neurotoxins of *Clostridium botulinum* of the types A, B, C, D, E, F or G or a mixture of two or more of said neurotoxins, **characterized in that** said neurotoxin or said mixture of said neurotoxins is free from the complex-forming proteins which naturally form complexes with said botulinum-neurotoxins, for the manufacture of a pharmaceutical preparation for the treatment of dystonia or of diseases of the nervous system in animal or human patients who have already formed neutralizing antibodies against botulinum-neurotoxin complexes.

2. Use of a pharmaceutical preparation comprising botulinum-neurotoxins of *Clostridium botulinum* of the types A, B, C, D, E, F or G or a mixture of two or more of said neurotoxins, **characterized in that** said neurotoxin or said mixture of said neurotoxins is free from the complex-forming proteins which naturally form complexes with said botulinum-neurotoxins, for the cosmetic treatment in animal or human patients who have already formed neutralizing antibodies against botulinum-neurotoxin complexes.

3. Use according to claim 1 or 2, **characterized in that** the person to be treated already exhibits neutralizing antibodies against a complex of *Clostridium botulinum* type A or B or a complex of *Clostridium botulinum* type A and B.

4. Use according to claim 2 or 3, **characterized in that** the cosmetic treatment is for treating hyperhidrosis.

5. Use according to one of claims 2 to 4, **characterized in that** the cosmetic treatment is for treating wrinkling.

6. Use according to claim 5, **characterized in that** the cosmetic treatment is for treating facial wrinkling.

7. Use according to claim 1 or 3, **characterized in that** the diseases of the nervous system or the dystonias are spasmodic torticollis, blepharospasm, spasticities such as equinus, hemifascial spasms, migraine, low back pain, cervical spine disorders or hypersalivation.

## Revendications

1. Utilisation des neurotoxines botuliques de *Clostridium botulinum* de types A, B, C, D, E, F ou G ou d'un mélange de deux ou plus desdites neurotoxines, **caractérisée en ce que** ladite neuro-toxine ou ledit mélange desdites neurotoxines est exempt(e) des protéines complexantes qui forment de manière naturelle des complexes avec lesdites neurotoxines botuliques, pour la fabrication d'une préparation pharmaceutique destinée au traitement de dystonies ou de maladies du système nerveux chez des patients animaux ou humains qui ont déjà produit des anticorps neutralisants contre les complexes de neurotoxine botulique.

2. Utilisationd'unepréparationpharmaceutique com-portant des neurotoxines botuliques de *Clostridium botulinum* de types A, B, C, D, E, F ou G ou d'un mélange de deux ou plus desdites neurotoxines, **caractérisée en ce que** ladite neurotoxine ou ledit mélange desdites neurotoxines est exempt(e) des protéines complexantes qui forment de manière naturelle des complexes avec lesdites neuro-toxines botuliques, pour le traitement cosmétique chez des patients animaux ou humains qui ont déjà produit des anticorps neutralisants contre les complexes de neurotoxine botulique.

3. Utilisation selon la revendication 1 ou 2, **caractérisée en ce que** la personne devant être traitée présente déjà des anticorps neutralisants contre un complexe de *Clostridium botulinum* type AouB,ou contre un complexe de *Clostridium botulinum* types A et B.

4. Utilisation selon la revendication 2 ou 3, **caractérisée en ce que** le traitement cosmétique sert à traiter l'hyperhidrose.

5. Utilisation selon l'une quelconque des revendi-cations 2 à4, **caractérisée en ce que** le traitement cosmétique sert à traiter la formation de rides.

6. Utilisation selon la revendication 5, **caracté-risée en ce que** le traitement cosmétique sert à traiter la formation de rides sur le visage.

7. Utilisation selon la revendication 1 ou 3, **caractérisée en ce que** les maladies du système nerveux ou les dystonies sont le torticolis spasmodique, le blépharospasme, les spasticités comme l'équinisme, les spasmes hémifaciaux, la migraine, les lombalgies, les troubles de la colonne cervicale ou l'hypersalivation.
